# EUROPÄISCHE PATENTSCHRIFT

(11) **EP 3 996 621 B1**
(45) Veröffentlichungstag und Bekanntmachung des Hinweises auf die Patenterteilung: **03.09.2025**
(21) Anmeldenummer: 20739971.8
(22) Anmeldetag: 10.07.2020
(51) Int. Cl.: A61B 90/00, G06M 1/00

(54) **VORRICHTUNG ZUR ERFASSUNG VON BETÄTIGUNGSZYKLEN EINES HANDBETÄTIGTEN INSTRUMENTS**
APPARATUS FOR DETECTING ACTIVATION CYCLES OF A MANUALLY CONTROLLED INSTRUMENT
DISPOSITIF DE DÉTECTION DE CYCLES D'ACTIONNEMENT D'UN INSTRUMENT À ACTIONNEMENT MANUEL

(30) Priorität: 11.07.2019 DE 102019118869
(43) Veröffentlichungstag der Anmeldung: 18.05.2022
(73) Patentinhaber: Aesculap AG, 78532 Tuttlingen (DE)
(72) Erfinder: HÖGERLE, Roland-Alois, 78532 Tuttlingen (DE); BÜRK, André, 78056 Villingen-Schwenningen (DE); KAHLER, Thomas-Erwin, 78606 Seitingen-Oberflacht (DE); LENZENHUBER, Frederick, 78532 Tuttlingen (DE)
(74) Vertreter: Winter, Brandl - Partnerschaft mbB
(86) Internationale Anmeldenummer: PCT/EP2020/069614
(87) Internationale Veröffentlichungsnummer: WO 2021/005224

(56) Entgegenhaltungen:
- WO-A1-2017/180460
- DE-A1- 3 414 467
- DE-T2- 69 435 028
- DE-T2- 69 813 533
- DE-U1- 202012 012 758
- DE-U1- 202013 100 949

## Beschreibung

Die Erfindung betrifft eine Vorrichtung und ein System zur Erfassung von Betätigungszyklen eines handbetätigten Instruments, und bezieht sich insbesondere auf eine Vorrichtung und ein System zum Erfassen, Zählen und/oder Dokumentieren von Betätigungszyklen bei Instrumenten im medizinischen Bereich, wie beispielsweise Scheren, Klemmen, Stanzen, laparoskopischen Instrumenten und dergleichen, sowie zur Bereitstellung daraus gewonnener relevanter Informationen zur Weiterverarbeitung.

### Hintergrund

Bislang ist es nicht möglich, Betätigungszyklen bei handbetriebenen, nicht mit Energie versorgten Instrumenten aus dem medizinischen Bereich in elektronischer und/oder digitaler Form zu erfassen und zu dokumentieren und auf dieser Grundlage gewonnene relevante Erkenntnisse weiterzuverarbeiten.

Aufgrund nicht verfügbarer Information wie beispielsweise solcher Betätigungszyklen ist es ferner nicht möglich, zumindest teilautomatisiert Aussagen zum allgemeinen Zustand eines handbetriebenen Produkts oder Instruments, über eine noch zu erwartende Lebensdauer oder ein bereits erreichtes Ende der Lebensdauer desselben, sein Leistungsvermögen und seine Eignung für nachfolgende Operationen (wie beispielsweise in einer Zweckbestimmung beschrieben) und/oder eine vorliegende Überlastung und gegebenenfalls eingetretene Schäden zu treffen.

Bislang sind dazu Tests und Sichtprüfungen durchzuführen, die zeitaufwändig und kostenintensiv sind und mangels erfasster Daten zudem jeweils einer Bandbreite der Beurteilung unterliegen, die unter Anwendung von Sicherheitsmargen dazu führen kann, dass Produkte und Instrumente sicherheitshalber (und damit zu früh) bereits ausgesondert werden, obschon diese bei einer Beurteilung auf Grundlage erfasster und insoweit gesicherter Benutzungsdaten gegebenenfalls noch länger verwendbar wären. Dies führt wiederum zu erhöhtem Beschaffungsaufwand mit damit verbundenen Kosten und zur Entsorgung von mehr Material, als einem tatsächlichen Benutzungszustand entsprechend notwendig wäre.

Ferner können mangels entsprechender Daten keine angepassten Dienstleistungen und individuell auf ihn abgestimmte Geschäftsmodelle für den Kunden erstellt werden, welches ebenfalls im Hinblick auf Beschaffungs- und Betriebskosten für den Kunden nicht optimal ist.

Außerdem kommt Fragestellungen bezüglich der Lebensdauer und der Beweisführung in etwa Reklamationsfällen zunehmend höhere Bedeutung und Wichtigkeit zu.

Weiterer relevanter Stand der Technik ist aus der DE 698 13 533 T2,
DE 20 2012 012 758 U1, DE 20 2013 100 949 U1, DE 34 14 467 A1,DE 694 35 028 T2 und WO 2017/180460 A1 bekannt.

### Kurzbeschreibung der Erfindung

Der Erfindung liegt als eine Aufgabe zugrunde, eine Vorrichtung und ein System zum Erfassen von Betätigungszyklen eines handbetriebenen, nicht mit Energie versorgten Produkts oder Instruments aus dem medizinischen Bereich bereitzustellen, mittels der ein Benutzer oder Kunde unmittelbar die Anzahl der bereits erfolgten Anwendungen erkennen kann, d.h. zählen, kann, wie oft das nicht energieversorgte Produkt oder Instrument bereits betätigt wurde, und ob es seine vorbestimmte Funktion noch erfüllen kann.

Erfindungsgemäß wird diese Aufgabe durch eine Vorrichtung mit den Merkmalen des Anspruchs 1 gelöst. Vorteilhafte Weiterbildungen der Erfindung sind Gegenstand der beigefügten Unteransprüche.

Die Anzahl von Betätigungszyklen ist in der Regel ein proportionales Maß für unter anderem den allgemeinen Zustand eines Instruments, dessen Lebensdauer bzw. Ende dessen Lebensdauer, dessen Leistungsvermögen und Eignung für eine bevorstehende Operation (wie in der Zweckbestimmung beschrieben) und dessen Überlastung und ggf. aufgetretene Produktschäden. Ein weiteres Maß können Aufbereitungszyklen des Instruments sein, die über einen auslesbaren Datenspeicher wie beispielsweise einen RFID-Chip, über NFC und BLE und dergleichen, in Verbindung mit einem geeigneten Datenerfassungssystem ermittelt werden können.

Der Erfindung liegt als eine allgemeine Idee zugrunde, eine als Betätigungszyklenzähler arbeitende Vorrichtung zum Erfassen von Betätigungszyklen eines nicht mit Energie versorgten, handbetriebenen medizinischen Instruments zu schaffen.

Der vorgenannte Betätigungszyklenzähler ist dazu angeordnet, Betätigungen des Instruments zu zählen und in Verbindung mit einem RFID-/ NFC-/BLE-Modul eine Möglichkeit zur Anzeige einer verbleibenden Lebensdauer des Instruments bereitzustellen. Die Erfassung erfolgt auf Grundlage einer ohne externe Energieversorgung arbeitenden Sensorvorrichtung, wobei das erfassbare bzw. zählbare und weiterverarbeitbare Betätigungssignal aus einer Handbewegung des Benutzers, beispielsweise eines Chirurgen, während der Anwendung des Instruments erzeugt wird.

Die grundlegende Idee besteht darin, dass ein vorbeigeführter Magnet (Neodym (Eisen-Bor), Samarium-Kobalt usw.) über eine Spule Energie induziert und jede einmalige Vorbeiführung des Magneten erkannt wird. Alternativ umfasst diese grundlegende Idee auch, dass bei einer Betätigung des Instruments über ein an diesem angeordnetes Druckerzeugungselement Druck auf ein an ebenfalls an dem Instrument angeordnetes Piezoelement ausgeübt wird, welches daraufhin eine Spannung erzeugt, und jede einmalige Spannungserzeugung erkannt wird. Jede der vorgenannten Aktionen erhöht einen Wert in einem Zähler um 1. Der jeweils aktuelle Zählerstand kann dann über eine Kombination aus beispielsweise einem Chip und einem Kern zuzüglich einer Spule, welche eine aktuelle Bauform von RFID / NFC-Chips abbildet, jederzeit über RFID / NFC Peripherie (z.B. Smartphones und dergleichen) ausgelesen werden.

In jedem Fall ist die Vorrichtung in Form einer Baugruppe "Betätigungszyklenzähler mit integrierter Spule zur Erkennung von Bewegung über Magnet" von einem geschlossenen Gehäuse (z.B. aus Glas, einer Keramik, eingespritzt in einem Kunststoffteil, integriert in einem Kunststoffteil usw.) umgeben, welches innenseitige Elektronik gegen Aufbereitungsmedien und Temperatur schützt. Ein Auslesen von Daten erfolgt über eine zusätzliche bzw. externe Vorrichtung mit einer entsprechenden Empfangsvorrichtung (beispielsweise ein Smartphone, ein Smart Tray, ein Tablet und dergleichen). Ausgelesene Daten können in einen externen Datenspeicher (beispielsweise ein Cloud-Dienst, eine Datenbank und dergleichen) geschrieben werden. Die erfindungsgemäße Lösung ist auf alle Gegenstände, Produkte und/oder Instrumente, welche über eine gewisse Zweiteiligkeit verfügen, übertragbar und skalierbar.

Es versteht sich, dass in Übereinstimmung mit der Form eines jeweiligen Instruments und/oder einem Anwendungsfall des Instruments Kombinationen aus Magneten, Spulen, Druckerzeugungselementen und Piezoelementen mit entsprechender Signalumsetzung und auch gegebenenfalls der Entfall einzelner der vorgenannten Elemente denkbar sind, solange final ein zur Zählung jedes Betätigungszyklus geeignetes, zählbares Signal generiert wird.

Es versteht sich ferner, dass die Erfindung in keiner Weise auf den medizinischen Bereich und dort verwendete Produkte, Systeme und/oder Instrumente beschränkt ist, sondern entsprechend modifizierte Konfigurationen und Modifikationen für zahlreiche weitere und/oder andere Produkte, denkbar und darstellbar sind.

Im Einzelnen wird die Aufgabe gelöst durch eine Vorrichtung zum Erfassen von Betätigungszyklen eines handbetätigten und handbetriebenen Instruments mit zumindest einem ersten und einem zweiten beweglichen Teil, wobei in einem Betätigungszyklus der erste und der zweite bewegliche Teil relativ zueinander bewegbar sind. Die Vorrichtung beinhaltet eine Energieerfassungsspule, die an einem des ersten und des zweiten beweglichen Teils angeordnet ist und dazu angeordnet ist, induktiv erzeugte Energie zu erfassen; einen Energieerzeugungsmagneten, der an dem anderen einem des ersten und des zweiten beweglichen Teils angeordnet ist dazu angeordnet ist, von der Energieerfassungsspule zu erfassende Energie induktiv zu erzeugen, wobei der Energieerzeugungsmagnet und die Energieerfassungsspule derart angeordnet sind, dass der Energieerzeugungsmagnet bei einer Bewegung des ersten und des zweiten beweglichen Teils relativ zueinander in einem Erfassungsbereich der Energieerfassungsspule relativ zu derselben beweglich ist und über die Energieerfassungsspule Energie in dieser induziert; und eine Speicher- und Verarbeitungseinrichtung, die einen Zähler aufweist und dazu angeordnet ist, bei jeder Relativbewegung ein auf der in der Energieerfassungsspule induzierten Energie basierendes Spannungssignal zu erfassen und bei jeder Erfassung des Spannungssignals den Zähler um Eins zu erhöhen und dadurch die Anzahl ausgeführter Betätigungszyklen des Instruments zu zählen.

In einer besonderen Ausführungsform wird das Instrument nicht mit Energie versorgt, d.h. als von einer äußeren Energiequelle autarkes Instrument ausgebildet (mit interner Energieumwandlungseinrichtung). In anderen Worten ausgedrückt wird das Instrument insbesondere nicht über eine externe Energiequelle versorgt. Anders ausgedrückt wird das Instrument ausschließlich über interne Energieumwandlungseinrichtungen mit Energie versorgt. Mit anderen Worten, das Instrument besitzt hierbei keine physische Verbindung, insbesondere kein Kabel zu einer externen, also außerhalb des Instruments, gelegenen Energiequelle.

In einer weiteren besonderen Ausführungsform kann das Instrument gemischt durch sowohl ein internes Glättungs-Puffermodul mit Ladeelektronik sowie durch eine Handbetätigung der zwei relativ zueinander bewegbaren Teile mit Energie versorgt werden. D.h. das Glättungs-Puffermodul und die Handbetätigung können als interne Energiequellen wirken.

In einer alternativen Ausführungsform kann das Instrument ausschließlich durch Handbetätigung der zwei relativ zueinander bewegbaren Teile mit Energie versorgt werden.

Bevorzugt ist in der Vorrichtung ein Magnetkern angeordnet und ist die Energieerfassungsspule auf den Magnetkern gewickelt. Der Magnetkern mit einer ihm zugeordneten Spule dient in Verbindung mit einem entsprechenden Chip oder Kombibaustein unter anderem zur Bereitstellung einer RFID/NFC-Funktionalität. Durch das direkte Wickeln der Energieerfassungsspule auf den Magnetkern kann vorteilhaft eine separate Energieerfassungsspule entfallen.

Bevorzugt ist in der Vorrichtung ein Magnetkern angeordnet, ist die Energieerfassungsspule separat von dem Magnetkern angeordnet und weist der Magnetkern eine weitere, auf ihn gewickelte Spule auf. Dedizierte Spulen am Magnetkern und für die Erfassung erhöhen vorteilhaft die Freiheitsgrade bei der Abstimmung der Vorrichtung auf einen jeweils vorgesehenen Anwendungsfall.

Bevorzugt ist der Energieerzeugungsmagnet ein Neodym- oder ein Samarium-Kobalt-Magnet und von einer Abschirmung derart umgeben, dass sein Magnetfeld in einer Wirkrichtung zur Energieerfassungsspule hin gerichtet und in anderen Richtungen als der Wirkrichtung abgeschwächt ist. Hierdurch wird vorteilhaft nur eine vorbestimmbare Wirkrichtung erreicht.

Bevorzugt ist der Energieerzeugungsmagnet in die Energieerfassungsspule einführbar und induziert Energie in derselben durch eine lineare Bewegung. Eine derartige Anordnung ist in bestimmten Anwendungsfällen vorteilhaft.

Bevorzugt ist der in die Energieerfassungsspule einführbare Energieerzeugungsmagnet an einem Schwingarm angeordnet und durch einen den Schwingarm mechanisch beaufschlagenden Körper in Schwingung versetzbar. Vorteilhaft kann auf diese Weise der Energieerzeugungsmagnet verlängert Energie erzeugen.

Bevorzugt ist der Energieerzeugungsmagnet in der Energieerfassungsspule drehbar angeordnet und induziert Energie in derselben durch eine Rotationsbewegung. Außerdem kann ein Übersetzungsgetriebe zur Drehzahlanpassung der Rotationsbewegung angeordnet sein, und/oder kann eine Schwungmasse zur Unterstützung der Aufrechterhaltung der Rotationsbewegung angeordnet sein.

Alternativ wird die Aufgabe im Einzelnen gelöst durch eine Vorrichtung zum Erfassen von Betätigungszyklen eines handbetätigten Instruments mit zumindest einem ersten und einem zweiten beweglichen Teil, wobei in einem Betätigungszyklus der erste und der zweite bewegliche Teil relativ zueinander bewegbar sind. Die Vorrichtung beinhaltet ein piezoelektrisches Element, das an einem des ersten und des zweiten beweglichen Teils angeordnet und dazu angeordnet ist, mit Druck, Zug und/oder Torsion beaufschlagt zu werden und dadurch eine Spannung aufzubauen; eine elektronische Schaltungsanordnung zum Betreiben des piezoelektrischen Elements; ein Druckerzeugungselement, das an dem anderen des ersten und des zweiten beweglichen Teils angeordnet ist und dazu angeordnet ist, das piezoelektrische Element mit Druck, Zug und/oder Torsion zu beaufschlagen; eine Energieerfassungsspule, die dazu angeordnet ist, die von dem piezoelektrischen Element aufgebaute Spannung in ein erfassbares Spannungssignal umzusetzen, wobei das piezoelektrische Element und das Druckerzeugungselement derart angeordnet sind, dass das Druckerzeugungselement bei einer Bewegung des ersten und des zweiten beweglichen Teils relativ zueinander den Druck, den Zug und/oder die Torsion auf das piezoelektrische Element ausübt und die dadurch von dem piezoelektrischen Element aufgebaute Spannung an die Energieerfassungsspule angelegt wird und dort das erfassbare Spannungssignal generiert; und eine Speicher- und Verarbeitungseinrichtung , die einen Zähler aufweist und dazu angeordnet ist, bei jeder Relativbewegung das in der Energieerfassungsspule generierte Spannungssignal zu erfassen und bei jeder Erfassung des Spannungssignals den Zähler um Eins zu erhöhen und dadurch die Anzahl ausgeführter Betätigungszyklen des Instruments zu zählen.

Bevorzugt ist auch in dieser alternativen Vorrichtung ein Magnetkern angeordnet, der Magnetkern eine auf ihn gewickelte Spule aufweist. Der Magnetkern mit einer ihm zugeordneten Spule dient in Verbindung mit einem entsprechenden Chip oder Kombibaustein unter anderem zur Bereitstellung einer RFID/NFC-Funktionalität. Durch das direkte Wickeln der Energieerfassungsspule auf den Magnetkern kann vorteilhaft eine separate Energieerfassungsspule entfallen. Es kann jedoch auch eine Energieerfassungsspule separat von dem Magnetkern angeordnet sein und der Magnetkern eine weitere, auf ihn gewickelte Spule aufweisen. Dedizierte Spulen am Magnetkern und für die Erfassung erhöhen vorteilhaft die Freiheitsgrade bei der Abstimmung der Vorrichtung auf einen jeweils vorgesehenen Anwendungsfall.

Bevorzugt weist die Speicher- und Verarbeitungseinrichtung ein EEPROM und eine integrierte Schaltung auf oder ist als ein Kombinationsbaustein ausgebildet, und stellt in Verbindung mit dem Magnetkern und der auf ihn gewickelten Spule eine von extern ansprechbare und/oder auslesbare RFID/NFC-Vorrichtung mit RFID/NFC-Funktionalität bereit.

Bevorzugt beinhaltet die Vorrichtung ein Glättungs-Puffermodul mit Ladeelektronik, wobei das Glättungs-Puffermodul einen Kondensator, einen PowerCap und/oder eine Batterie aufweist und zur Stützung der Speicher- und Verarbeitungseinrichtung bereitgestellt ist. Hierdurch wird vorteilhaft erreicht, dass die Baugruppe um ein zusätzliches Glättungs-Puffermodul (Kondensator, Powercap, Batterie, usw.) mit Ladeelektronik erweitert wird, um beispielsweise den Chip oder Kombibaustein länger empfangbar zu halten.

Bevorzugt besteht ein den ersten beweglichen Teil und den zweiten beweglichen Teil verbindendes Drehgelenk zumindest teilweise aus einem Piezoelement, das dazu angeordnet ist, die Vorrichtung vermittels Druck und Torsion mit Energie zu speisen.

Bevorzugt ist das Piezoelement in Form eines Gehäuses ausgebildet und dazu angeordnet, die weiteren Komponenten der Vorrichtung dicht zu umschließen und bei Beaufschlagung mit Vibration, Schock oder Druck Energie in der Vorrichtung zu induzieren.

### Kurze Beschreibung der Figuren

Die Erfindung wird nachstehend unter Bezugnahme auf die beigefügte Zeichnung näher beschrieben. Es zeigen:
Fig. 1 eine schematische Darstellung einer Vorrichtung zum Erfassen von Betätigungszyklen eines handbetätigten Instruments gemäß einem ersten Ausführungsbeispiel;
Fig. 2 eine schematische Darstellung einer Vorrichtung zum Erfassen von Betätigungszyklen eines handbetätigten Instruments gemäß einem zweiten Ausführungsbeispiel;
Fig. 3 eine schematische Darstellung einer Vorrichtung zum Erfassen von Betätigungszyklen eines handbetätigten Instruments gemäß einem dritten Ausführungsbeispiel;
Fig. 4 eine schematische Darstellung einer Vorrichtung zum Erfassen von Betätigungszyklen eines handbetätigten Instruments gemäß einem vierten Ausführungsbeispiel;
Fig. 5 eine schematische Darstellung eines von einer Abschirmung umgegebenen Magneten, der in Ausführungsbeispielen als ein Energieerzeugungsmagnet verwendbar ist;
Fig. 6 eine schematische Darstellung einer Vorrichtung zum Erfassen von Betätigungszyklen eines handbetätigten Instruments gemäß einem fünften Ausführungsbeispiel;
Fig. 7 eine schematische Darstellung einer Vorrichtung zum Erfassen von Betätigungszyklen eines handbetätigten Instruments gemäß einem sechsten Ausführungsbeispiel;
Fig. 8 eine schematische Darstellung einer Vorrichtung zum Erfassen von Betätigungszyklen eines handbetätigten Instruments gemäß einem siebten Ausführungsbeispiel;
Fig. 9 eine schematische Darstellung einer Vorrichtung zum Erfassen von Betätigungszyklen eines handbetätigten Instruments gemäß einem achten Ausführungsbeispiel;
Fig. 10 eine schematische Darstellung einer Vorrichtung zum Erfassen von Betätigungszyklen eines handbetätigten Instruments in einer alternativen Ausführungsform gemäß einem neunten Ausführungsbeispiel;
Fig. 11 eine schematische Darstellung eines in Ausführungsbeispielen verwendbaren Piezoelements mit zugeordneter Energieverwaltung; und
Fig. 12 eine schematische Darstellung einer Vorrichtung zum Erfassen von Betätigungszyklen eines handbetätigten Instruments gemäß einem zehnten Ausführungsbeispiel.

In den Figuren bezeichnen gleiche Bezugszeichen gleiche oder zumindest äquivalente Teile und Komponenten. Zweckmäßig wird insoweit eine mehrfach redundante Beschreibung solcher Teile und Komponenten weggelassen.

### Detaillierte Beschreibung bevorzugter Ausführungsbeispiele

Bevorzugte Ausführungsbeispiele einer hierin beschriebenen Vorrichtung zum Erfassen, Zählen und/oder Dokumentieren von Betätigungszyklen eines handbetriebenen, nicht mit Energie versorgten Produkts oder Instruments (nachstehend auch als Betätigungszyklenzähler bezeichnet) werden nachstehend unter Bezugnahme auf die beigefügten Figuren beschrieben.

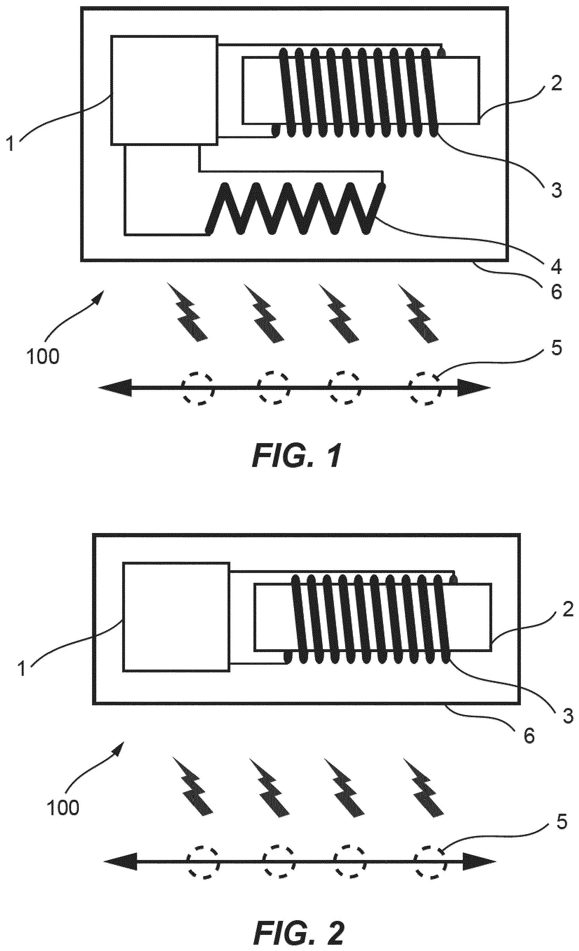

Fig. 1 zeigt eine schematische Darstellung der Vorrichtung zum Erfassen von Betätigungszyklen eines handbetätigten Instruments bzw. eines Betätigungszyklenzählers 100 gemäß einem ersten Ausführungsbeispiel.

Gemäß Fig. 1 beinhaltet der Betätigungszyklenzähler 100 eine Speicher- und Verarbeitungseinrichtung 1, die in Form eines EEPROMS, eines ICs mit einer integrierten Schaltungsanordnung, eines Chips oder eines Kombinationsbausteins mit einem oder mehreren der vorgenannten Elemente bereitgestellt sein kann und allgemein zur Speicherung und Verarbeitung von in dem Betätigungszyklenzähler erfassten, verarbeiteten oder anderweitig bereitgestellten Daten, Werten, Signalen usw. angeordnet ist. Ferner beinhaltet der Betätigungszyklenzähler 100 einen Magnetkern 2 mit einer direkt auf ihn gewickelten Spulenanordnung bzw. Spule 3. Die Spule 3 ist an ihren beiden Enden jeweils mit entsprechenden Eingängen an der Speicher- und Verarbeitungseinrichtung 1 verbunden. Außerdem weist der Betätigungszyklenzähler 100 eine Energieerfassungsspule 4 zur Bereitstellung eines Signals, beispielsweise eines Spannungssignals, auf, die zur Realisierung einer Zählfunktion in der Speicher- und Verarbeitungseinrichtung 1 an ihren Enden ebenfalls mit entsprechenden Eingängen der Speicher- und Verarbeitungseinrichtung 1 verbunden ist. In dem ersten Ausführungsbeispiel ist die Energieerfassungsspule 4 separat von der Spule 3 des Magnetkerns 2 angeordnet. Ein dicht abschließendes Gehäuse 6 umgibt die vorgenannten Komponenten oder Baugruppen 2 bis 4 des Betätigungszyklenzählers 100.

Darüber hinaus beinhaltet der Betätigungszyklenzähler 100 einen Energieerzeugungsmagneten 5, der sich relativ zu der Energieerfassungsspule 4 beweglich außerhalb des Gehäuses 6 befindet und aus beispielsweise Neodym (Eisen-Bor), Samarium-Kobalt oder dergleichen bestehen kann. Um das Magnetfeld gerichtet zur Spule zu erhalten und andere Bereiche mit dem Energieerfassungsmagneten 5 nicht zu beeinflussen, ist der Energieerfassungsmagnet 5 von einer noch zu beschreibenden Abschirmung umgeben. Somit wird nur eine Wirkrichtung erreicht.

Der Betätigungszyklenzähler 100 weist mit anderen Worten die Energieerfassungsspule 4, die an einem eines ersten und eines zweiten beweglichen Teils eines zumindest zweiteiligen Produkts oder Instruments angeordnet ist und dazu angeordnet ist, induktiv erzeugte Energie zu erfassen, und den Energieerzeugungsmagneten 5, der an dem anderen einen des ersten und des zweiten beweglichen Teils des zumindest zweiteiligen Produkts oder Instruments angeordnet ist und dazu angeordnet ist, von der Energieerfassungsspule 4 zu erfassende Energie induktiv zu erzeugen, auf.

Das heißt, an einem der ersten und zweiten beweglichen Teile ist der die Komponenten 1 bis 4 und das Gehäuse 6 umfassende Teil des Betätigungszyklenzählers 100 angeordnet, und an dem anderen der ersten und zweiten beweglichen Teile ist der Energieerzeugungsmagnet 5 angeordnet. Der Energieerzeugungsmagnet 5 und die Energieerfassungsspule 4 sind derart angeordnet, dass der Energieerzeugungsmagnet 5 bei einer Bewegung des ersten und des zweiten beweglichen Teils relativ zueinander in einem Erfassungsbereich der Energieerfassungsspule 4 relativ zu derselben beweglich ist und/oder an dieser vorbeigeführt wird und dabei über die Energieerfassungsspule 4 Energie in dieser induziert. Diese (hier lineare) Relativbewegung ist in Fig. 1 mit einem an verschiedenen Positionen entlang eines Doppelpfeils mit durchbrochener Linie angedeuteten Energieerzeugungsmagneten 5 und an den verschiedenen Positionen zu der Energieerfassungsspule 4 hin weisenden Spannungspfeilsymbolen veranschaulicht. Es versteht sich, dass die Relativbewegung nicht auf eine lineare Bewegung beschränkt ist, und dass auch andere Relativbewegungen geeignet sind, die erwünschte Energie in der Energieerfassungsspule 4 zu generieren.

Die Speicher- und Verarbeitungseinrichtung 1 weist einen Zähler (nicht gezeigt) auf und ist dazu angeordnet, bei jeder Relativbewegung ein auf der in der Energieerfassungsspule 4 induzierten Energie basierendes Spannungssignal zu erfassen und bei jeder Erfassung des Spannungssignals den Zähler um Eins (1) zu erhöhen und dadurch die Anzahl ausgeführter Betätigungszyklen des Instruments zu zählen.

Der Betätigungszyklenzähler 100 mit integrierter Energieerfassungsspule 4 zur Erkennung von Bewegung über den Energieerzeugungsmagneten 5 ist auf Gegenstände, Produkte, Instrumente und dergleichen übertragbar und skalierbar, sofern diese über eine gewisse Zweiteiligkeit verfügen, d.h. zumindest einen ersten beweglichen Teil und einen zweiten beweglichen Teil aufweisen, die relativ zueinander beweglich sind.

Fig. 2 zeigt eine schematische Darstellung der Vorrichtung zum Erfassen von Betätigungszyklen eines handbetätigten Instruments gemäß einem zweiten Ausführungsbeispiel.

Während in der Vorrichtung gemäß dem in Fig. 1 dargestellten ersten Ausführungsbeispiel der Magnetkern 2 mit der für ihn dedizierten Spule 3 angeordnet und die Energieerfassungsspule 4 separat von dem Magnetkern 2 angeordnet sind, ist in dem in Fig. 2 gezeigten zweiten Ausführungsbeispiel die Energieerfassungsspule direkt auf den Magnetkern 2 gewickelt.

Der Magnetkern 2 mit einer ihm zugeordneten Spule dient in Verbindung mit einem entsprechenden Chip oder Kombibaustein unter anderem zur Bereitstellung einer RFID/NFC-Funktionalität. Durch das direkte Wickeln der Energieerfassungsspule auf den Magnetkern kann vorteilhaft eine separate Energieerfassungsspule 4 entfallen. Mit anderen Worten wird die Funktion der separaten Energieerfassungsspule 4 in dem zweiten Ausführungsbeispiel von der Spule 3 des Magnetkerns 2 mit übernommen.

Die übrigen Komponenten und die Funktionsweise des in Fig. 2 gezeigten zweiten Ausführungsbeispiels des Betätigungszyklenzählers 100 entsprechen den Komponenten und der Funktionsweise des in Fig. 1 dargestellten ersten Ausführungsbeispiels des Betätigungszyklenzählers 100, so dass eine diesbezüglich redundante Beschreibung zweckmäßig entfallen kann.

Bevorzugt ist der Energieerzeugungsmagnet ein Neodym- oder ein Samarium-Kobalt-Magnet und von einer Abschirmung derart umgeben, dass sein Magnetfeld in einer Wirkrichtung zur Energieerfassungsspule hin gerichtet und in anderen Richtungen als der Wirkrichtung abgeschwächt ist. Hierdurch wird vorteilhaft nur eine vorbestimmbare Wirkrichtung erreicht.

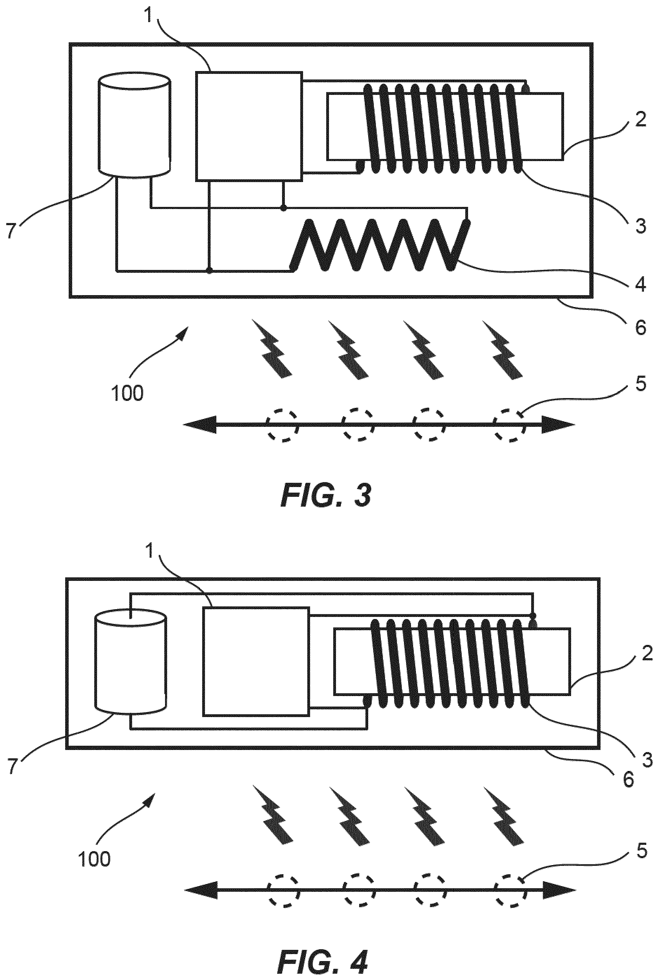

Fig. 3 zeigt eine schematische Darstellung der Vorrichtung zum Erfassen von Betätigungszyklen eines handbetätigten Instruments gemäß einem dritten Ausführungsbeispiel, und Fig. 4 zeigt eine schematische Darstellung der Vorrichtung zum Erfassen von Betätigungszyklen eines handbetätigten Instruments gemäß einem vierten Ausführungsbeispiel.

Das dritte und das vierte Ausführungsbeispiel entsprechen in Aufbau und Funktionsweise jeweils dem vorstehend beschriebenen ersten bzw. zweiten Ausführungsbeispiel, mit der Ausnahme, dass in dem dritten und dem vierten Ausführungsbeispiel jeweils ein Glättungs-Puffermodul 7 (das einen Kondensator, beispielsweise einen als Varianz dienenden Kondensator, einen Powercap, eine Batterie, usw. beinhalten kann) mit einer Ladeelektronik (nicht gezeigt) zusätzlich angeordnet ist, um die Speicher- und Verarbeitungseinrichtung (den Chip) 1 länger "empfangbar" zu halten. Das Glättungs-Puffermodul 7 bildet somit einen zusätzlichen Energiespeicher. In diesem Kontext kann die Energieerfassungsspule 3 auch als eine Induktionsspule ausgeführt sein, die ein induktives (kabelloses) Bestromen des Glättungs-Puffermoduls 7 ermöglicht. Der benötigte Energieeintrag kann hierbei durch beispielsweise ein Induktiv-Ladegerät auf beispielsweise einem Operationstisch erfolgen

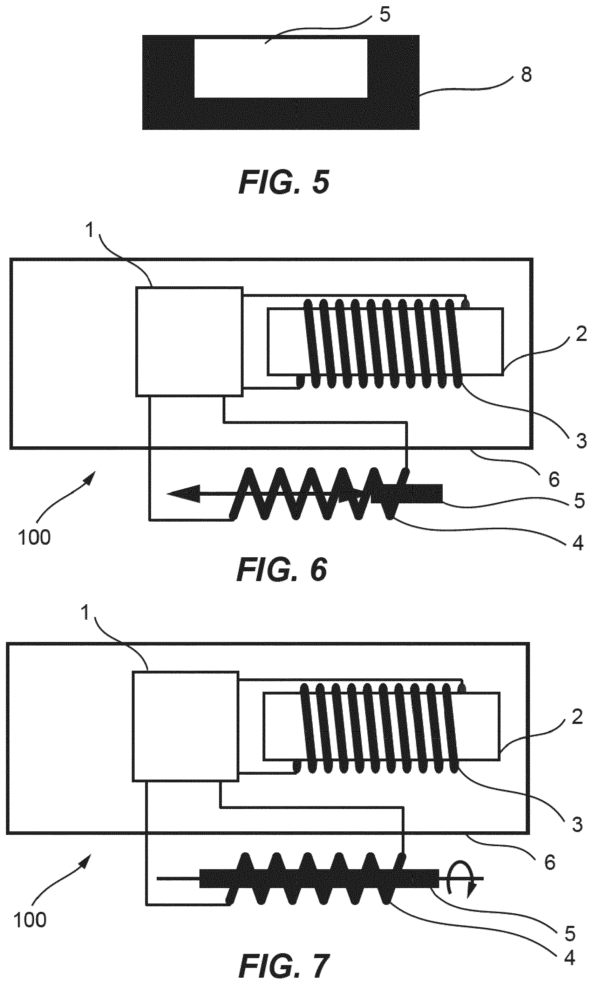

Fig. 5 zeigt eine schematische Darstellung eines von einer Abschirmung umgegebenen Energieerfassungsmagneten 5, der in Ausführungsbeispielen des Betätigungszyklenzählers 100 als der Energieerzeugungsmagnet 5 verwendbar ist. Um das Magnetfeld gerichtet zur Energieerfassungsspule zu erhalten und andere Bereiche mit dem Energieerzeugungsmagneten 5 nicht zu beeinflussen, ist der Energieerzeugungsmagnet 5 derart mit einer Abschirmung 8 umgeben, dass nur eine Wirkrichtung erreicht wird.

Fig. 6 zeigt eine schematische Darstellung der Vorrichtung zum Erfassen von Betätigungszyklen eines handbetätigten Instruments gemäß einem fünften Ausführungsbeispiel.

Das fünfte Ausführungsbeispiel entspricht in Aufbau und Funktionsweise dem vorstehend beschriebenen ersten Ausführungsbeispiel, mit der Ausnahme, dass die Energieerfassungsspule 4 so angeordnet ist, dass ein in die Energieerfassungsspule 5 eingeführter Energieerzeugungsmagnet die Energie durch eine lineare (Hin- und Her-) Bewegung in der Energieerfassungsspule 4 induziert.

Fig. 7 zeigt eine schematische Darstellung der Vorrichtung zum Erfassen von Betätigungszyklen eines handbetätigten Instruments gemäß einem sechsten Ausführungsbeispiel.

Das sechste Ausführungsbeispiel entspricht in Aufbau und Funktionsweise dem vorstehend beschriebenen ersten Ausführungsbeispiel, mit der Ausnahme, dass die Energieerfassungsspule 4 derart ausgebildet ist, dass ein in der Energieerfassungsspule 4 drehbar angeordneter Energieerzeugungsmagnet 5 Energie durch eine Rotationsbewegung induziert. Zur Anpassung an höhere Drehzahlen kann darüber hinaus ein Übersetzungsgetriebe (nicht gezeigt) für höhere Drehzahlen angeordnet sein.

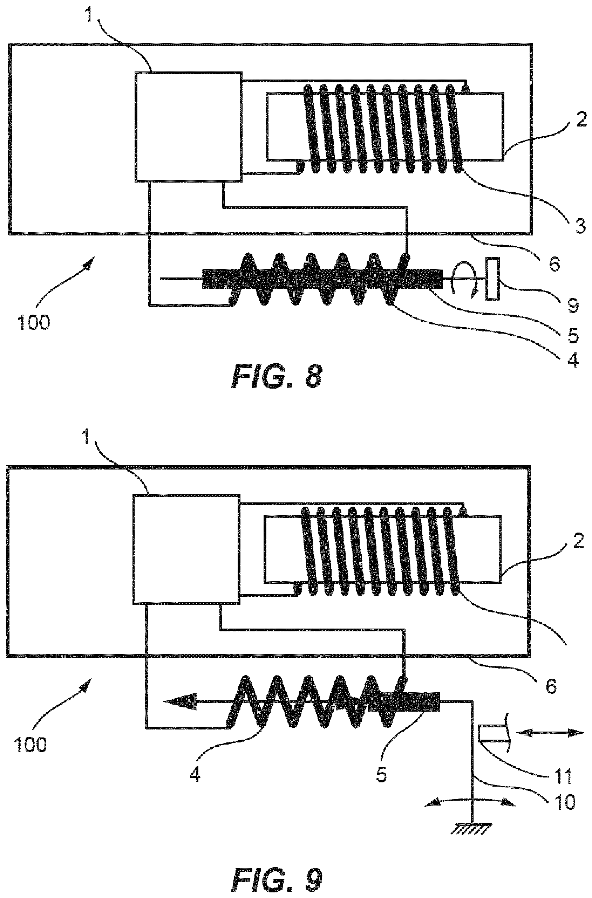

Fig. 8 zeigt eine schematische Darstellung einer Vorrichtung zum Erfassen von Betätigungszyklen eines handbetätigten Instruments gemäß einem siebten Ausführungsbeispiel. Das siebte Ausführungsbeispiel entspricht in Aufbau und Funktionsweise dem vorstehend beschriebenen sechsten Ausführungsbeispiel, mit der Ausnahme, dass in einer Modifikation desselben zusätzlich eine Schwungmasse 9, beispielsweise ein Schwungrad, angeordnet sein kann, um die Drehbewegung des Energieerzeugungsmagneten 5 länger aufrecht zu erhalten.

Fig. 9 zeigt eine schematische Darstellung einer Vorrichtung zum Erfassen von Betätigungszyklen eines handbetätigten Instruments gemäß einem achten Ausführungsbeispiel. In dem achten Ausführungsbeispiel ist in einer weiteren Bauform die Energieerfassungsspule 4 derart angeordnet, dass ein in der Energieerfassungsspule 4 an einem Schwingarm 10 angeordneter Energieerfassungsmagnet 5 durch einen den Schwingarm 10 beaufschlagenden, d.h. betätigenden, Körper 11, der beispielsweise in Form eines stiftförmigen Betätigungselements ausgebildet sein kann, in Schwingung versetzt wird und dadurch verlängert Energie erzeugen kann.

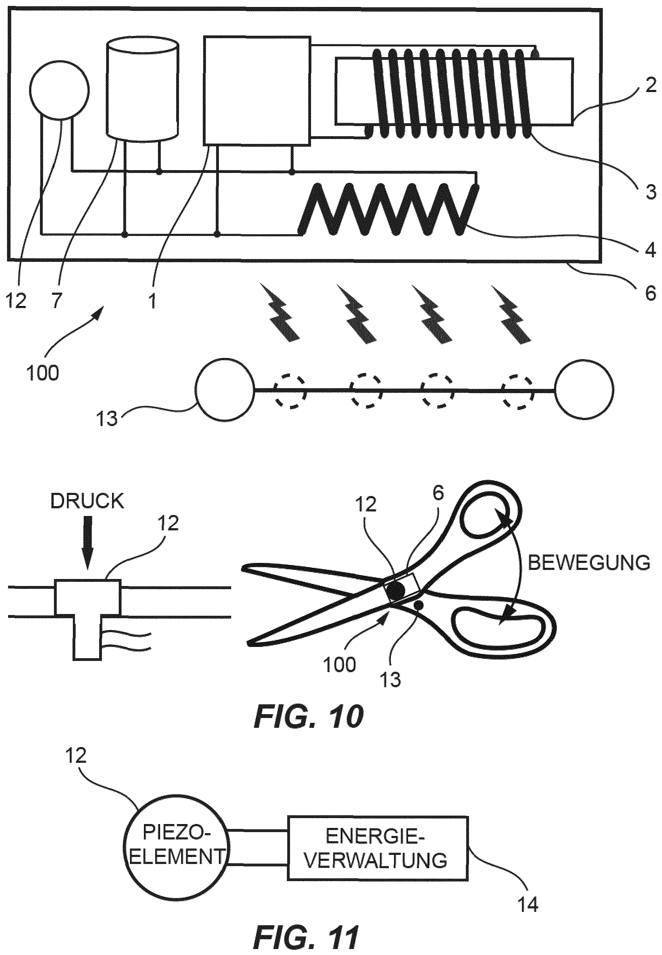

Fig. 10 zeigt eine schematische Darstellung einer Vorrichtung zum Erfassen von Betätigungszyklen eines handbetätigten Instruments in einer alternativen Ausführungsform gemäß einem neunten Ausführungsbeispiel.

In der alternativen Ausführungsform gemäß Fig. 10 wird als Energiegewinnungseinrichtung anstelle des Energieerzeugungsmagneten 5 und Zähleinrichtung ein piezoelektrisches Element oder Piezoelement 12 genutzt, welches (elektrische) Spannung aufbaut, sofern es von einem Druckerzeugungselement 13 mit Druck, Zug und/oder Torsion beaufschlagt wird. Ebenso kann es (elektrische) Spannung durch Einwirkung einer Vibration und eines Schocks aufbauen. Durch Druck, Zug, oder Torsion entsteht somit eine (elektrische) Spannung beim Schließen des (zumindest zweiteiligen) Instruments über die Relativbewegung des ersten beweglichen Teils und des zweiten beweglichen Teils desselben.

Wie im unteren Teil der Fig. 10 vereinfacht dargestellt ist, kann beispielsweise das Piezoelement 12 im Bereich eines Drehgelenks eines Instruments (beispielsweise einer Schere) mit zwei relativ zueinander beweglichen Teilen an einem der beiden relativ zueinander beweglichen Teilen angeordnet, beispielsweise in einen der Schenkel der Schere eingelassen, sein und kann das Druckerzeugungselement 13 an dem anderen der beiden relativ zueinander beweglichen Teile angeordnet, beispielsweise in den anderen der Schenkel der Schere, eingelassen sein. Wird die Schere geschlossen, drückt das Druckerzeugungselement 13 gegen das Piezoelement 12, welches daraufhin eine Spannung aufbaut. Diese Spannung wird in der Energieerfassungsspule 4 erfasst und in ein von der Speicher- und Verarbeitungseinrichtung 1 messbares bzw. weiterverarbeitbares und schließlich zählbares Signal umgesetzt.

In einer weiteren Modifikation kann beispielsweise ein solches Drehgelenk zumindest teilweise oder ganz aus einem Piezoelement 12 bestehen, welches somit bedingt durch Druck und Torsion die Vorrichtung mit Energie speist.

Das Piezoelement 12 muss mit einer geeigneten elektrischen oder elektronischen Schaltungsanordnung 14 für eine Energieverwaltung bzw. ein Energiemanagement betrieben werden. Diese Schaltungsanordnung 14 ist in Fig. 11 schematisch gezeigt und ist notwendig, um ein Bestromen des Piezoelements 12 durch das Glättungs- und Puffermodul 7 (beispielsweise durch einen darin enthaltenen, als Varianz dienender Kondensator) zu verhindern. Ebenso ermöglicht diese Schaltungsanordnung 14 das Laden eines solchen Kondensators.

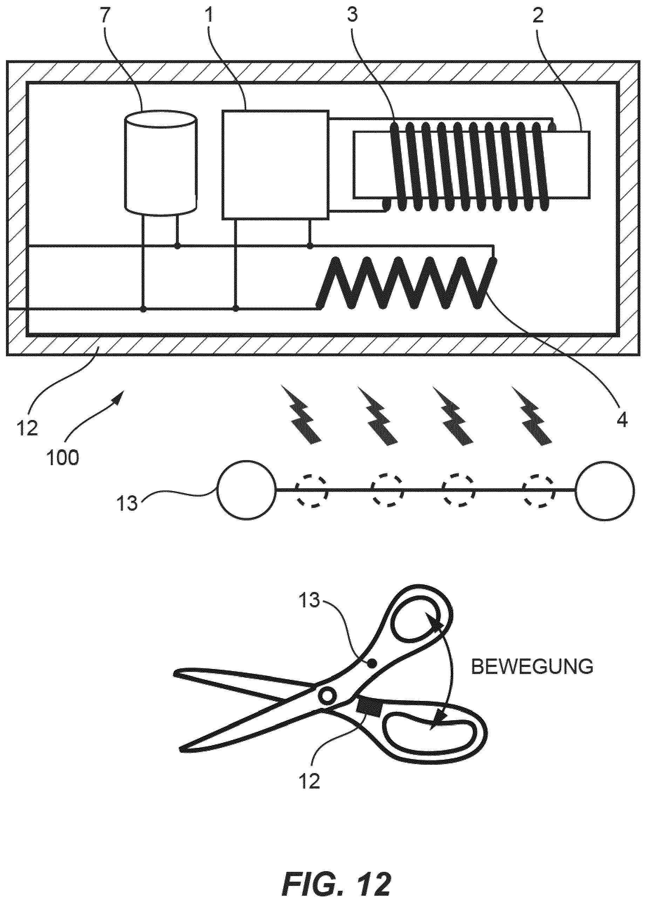

Fig. 12 eine schematische Darstellung einer Vorrichtung zum Erfassen von Betätigungszyklen eines handbetätigten Instruments gemäß einem zehnten Ausführungsbeispiel.

Gemäß Fig. 12 ist in einer weiteren Modifikation das Piezoelement 12 als Gehäuse ausgebildet, das die Vorrichtung bzw. Baugruppe als Gehäuse dicht umschließt und dann, wenn es von außen mit einer Vibration, einem Schock oder Schlag oder einem Druck beaufschlagt wird, Energie in die Baugruppe induziert.

Wie vorstehend beschrieben wurde, sind bei einer Vorrichtung zum Erfassen von Betätigungszyklen eines handbetätigten Instruments in einem Betätigungszyklus ein erster und ein zweiter beweglicher Teil des Instruments relativ zueinander bewegbar. Eine Energieerfassungsspule an einem des ersten und des zweiten beweglichen Teils erfasst induktiv erzeugte Energie. Ein Energieerzeugungsmagnet an dem anderen einem des ersten und des zweiten beweglichen Teils erzeugt von der Energieerfassungsspule zu erfassende Energie induktiv. Der Energieerzeugungsmagnet ist bei einer Bewegung des ersten und des zweiten beweglichen Teils relativ zueinander in einem Erfassungsbereich der Energieerfassungsspule relativ zu derselben beweglich und induziert in dieser Energie. Eine Speicher- und Verarbeitungseinrichtung weist einen Zähler auf, erfasst bei jeder Relativbewegung ein auf der induzierten Energie basierendes Spannungssignal, erhöht bei jeder Erfassung des Spannungssignals den Zähler um Eins und zählt dadurch die Anzahl ausgeführter Betätigungszyklen des Instruments. In einer alternativen Ausführungsform wird das Spannungssignal unter Verwendung einer von einem piezoelektrischen Element aufgebauten Spannung generiert.

Es versteht sich, dass die Erfindung nicht auf die vorstehend beschriebenen Ausführungsbeispiele beschränkt ist, sondern sich für den Fachmann Änderungen, Modifikationen, Kombinationen und äquivalente Anordnungen im Rahmen des anspruchsgemäß definierten Schutzumfangs ohne weiteres ergeben.

## Patentansprüche

1. Handbetätigtes und handbetriebenes, medizinisches Instrument mit einer Vorrichtung (100) zum Erfassen von Betätigungszyklen des handbetätigten Instruments mit zumindest einem ersten und einem zweiten beweglichen Teil, wobei in einem Betätigungszyklus der erste und der zweite bewegliche Teil relativ zueinander bewegbar sind, wobei die Vorrichtung (100) Folgendes umfasst:
eine Energieerfassungsspule (3, 4), die an einem des ersten und des zweiten beweglichen Teils angeordnet ist und dazu angeordnet ist, induktiv erzeugte Energie zu erfassen;
einen Energieerzeugungsmagneten (5), der an dem anderen einem des ersten und des zweiten beweglichen Teils angeordnet ist und dazu angeordnet ist, von der Energieerfassungsspule (3, 4) zu erfassende Energie induktiv zu erzeugen,
wobei der Energieerzeugungsmagnet (5) und die Energieerfassungsspule (3, 4) derart angeordnet sind, dass der Energieerzeugungsmagnet (5) bei einer Bewegung des ersten und des zweiten beweglichen Teils relativ zueinander in einem Erfassungsbereich der Energieerfassungsspule (3, 4) relativ zu derselben beweglich ist und über die Energieerfassungsspule (3, 4) Energie in dieser induziert; und
eine Speicher- und Verarbeitungseinrichtung (1), die einen Zähler aufweist und dazu angeordnet ist, bei jeder Relativbewegung ein auf der in der Energieerfassungsspule (3, 4) induzierten Energie basierendes Spannungssignal zu erfassen und bei jeder Erfassung des Spannungssignals den Zähler um Eins zu erhöhen und dadurch die Anzahl ausgeführter Betätigungszyklen des Instruments zu zählen.

2. Handbetätigtes und handbetriebenes, medizinisches Instrument mit einer Vorrichtung (100) nach Anspruch 1, **dadurch gekennzeichnet, dass** das Instrument nicht mit Energie, insbesondere nicht mit Energie einer externen Energiequelle, versorgt ist.

3. Handbetätigtes und handbetriebenes, medizinisches Instrument mit einer Vorrichtung nach Anspruch 1, **dadurch gekennzeichnet, dass** an die Vorrichtung (100) ein Magnetkern (2) angeordnet ist und die Energieerfassungsspule (3) auf den Magnetkern (2) gewickelt ist.

4. Handbetätigtes und handbetriebenes, medizinisches Instrument mit einer Vorrichtung (100) nach Anspruch 1, **dadurch gekennzeichnet, dass** an die Vorrichtung (100) ein Magnetkern (2) angeordnet ist, die Energieerfassungsspule (4) separat von dem Magnetkern (2) angeordnet ist und der Magnetkern (2) eine weitere, auf ihn gewickelte Spule (3) aufweist.

5. Handbetätigtes und handbetriebenes, medizinisches Instrument mit einer Vorrichtung (100) nach einem der Ansprüche 1 bis 4, **dadurch gekennzeichnet, dass** der Energieerzeugungsmagnet (5) ein Neodym- oder ein Samarium-Kobalt-Magnet ist und von einer Abschirmung (8) derart umgeben ist, dass sein Magnetfeld in einer Wirkrichtung zur Energieerfassungsspule (4) hin gerichtet und in anderen Richtungen als der Wirkrichtung abgeschwächt ist.

6. Handbetätigtes und handbetriebenes, medizinisches Instrument mit einer Vorrichtung (100) nach einem der Ansprüche 1 bis 5, **dadurch gekennzeichnet, dass** der Energieerzeugungsmagnet (5) in die Energieerfassungsspule (4) einführbar ist und Energie in derselben durch eine lineare Bewegung induziert.

7. Handbetätigtes und handbetriebenes, medizinisches Instrument mit einer Vorrichtung (100) nach Anspruch 6, **dadurch gekennzeichnet, dass** der in die Energieerfassungsspule (4) einführbare Energieerzeugungsmagnet (5) an einem Schwingarm (10) angeordnet und durch einen den Schwingarm (10) mechanisch beaufschlagenden Körper (11) in Schwingung versetzbar ist.

8. Handbetätigtes und handbetriebenes, medizinisches Instrument mit einer Vorrichtung (100) nach einem der Ansprüche 1 bis 5, **dadurch gekennzeichnet, dass** der Energieerzeugungsmagnet (5) in der Energieerfassungsspule (4) drehbar angeordnet ist und Energie in derselben durch eine Rotationsbewegung induziert.

9. Handbetätigtes und handbetriebenes, medizinisches Instrument mit einer Vorrichtung (100) nach Anspruch 8, **dadurch gekennzeichnet, dass** ein Übersetzungsgetriebe zur Drehzahlanpassung der Rotationsbewegung angeordnet ist.

10. Handbetätigtes und handbetriebenes, medizinisches Instrument mit einer Vorrichtung (100) nach Anspruch 8 oder 9, **dadurch gekennzeichnet, dass** eine Schwungmasse (9) zur Unterstützung der Aufrechterhaltung der Rotationsbewegung angeordnet ist.

11. Handbetätigtes und handbetriebenes, medizinisches Instrument mit einer Vorrichtung (100) nach einem der Ansprüche 3 bis 10, **dadurch gekennzeichnet, dass** die Speicher- und Verarbeitungseinrichtung (1) ein EEPROM und eine integrierte Schaltung aufweist oder als ein Kombinationsbaustein ausgebildet ist, und in Verbindung mit dem Magnetkern (2) und der auf ihn gewickelten Spule (3) eine von extern ansprechbare und/oder auslesbare RFID/NFC-Vorrichtung mit RFID/NFC-Funktionalität bereitstellt.

12. Handbetätigtes und handbetriebenes, medizinisches Instrument mit einer Vorrichtung (100) nach einem der vorangehenden Ansprüche, **gekennzeichnet durch** ein Glättungs-Puffermodul (7) mit Ladeelektronik, wobei das Glättungs-Puffermodul (7) einen Kondensator, einen PowerCap und/oder eine Batterie aufweist und zur Stützung der Speicher- und Verarbeitungseinrichtung (1) bereitgestellt ist.

## Claims

1. A hand-operated and hand-powered, medical instrument with an apparatus (100) for detecting activation cycles of the hand-operated instrument with at least a first and a second movable part, wherein in an activation cycle, the first and the second movable part are movable relative to each other, wherein the apparatus (100) comprises:
an energy detection coil (3, 4) arranged on one of the first and second movable parts and arranged to detect inductively generated energy;
an energy-generating magnet (5) arranged on the other one of the first and second movable parts and arranged to inductively generate energy to be detected by the energy detection coil (3, 4),
wherein the energy-generating magnet (5) and the energy detection coil (3, 4) are arranged such that, upon movement of the first and second movable parts relative to each other, the energy-generating magnet (5) is movable within a detection range of the energy detection coil (3, 4) relative thereto and induces energy therein via the energy detection coil (3, 4); and
a memory and processing device (1) having a counter and arranged to detect, at each relative movement, a voltage signal based on the energy induced in the energy detection coil (3, 4) and to increment the counter by one at each detection of the voltage signal, thereby counting the number of executed activation cycles of the instrument.

2. The hand-operated and hand-powered, medical instrument with an apparatus (100) according to claim 1, **characterized in that** the instrument is not supplied with energy, in particular not with energy from an external energy source.

3. The hand-operated and hand-powered, medical instrument with an apparatus according to claim 1, **characterized in that** a magnetic core (2) is arranged at the apparatus (100) and the energy detection coil (3) is wound on the magnetic core (2).

4. The hand-operated and hand-powered, medical instrument with an apparatus (100) according to claim 1, **characterized in that** a magnetic core (2) is arranged at the apparatus (100), the energy detection coil (4) is arranged separately from the magnetic core (2) and the magnetic core (2) has a further coil (3) wound onto it.

5. The hand-operated and hand-powered, medical instrument with an apparatus (100) according to one of claims 1 to 4, **characterized in that** the energy-generating magnet (5) is a neodymium or a samarium-cobalt magnet and is surrounded by a shield (8) in such a way that its magnetic field is directed in an effective direction towards the energy detection coil (4) and is attenuated in directions other than the effective direction.

6. The hand-operated and hand-powered, medical instrument with an apparatus (100) according to one of claims 1 to 5, **characterized in that** the energy-generating magnet (5) is insertable into the energy detection coil (4) and induces energy therein by a linear movement.

7. The hand-operated and hand-powered, medical instrument with an apparatus (100) according to claim 6, **characterized in that** the energy-generating magnet (5) that can be introduced into the energy detection coil (4) is arranged on a pivot arm (10) and can be caused to oscillate by a body (11) mechanically acting on the pivot arm (10).

8. The hand-operated and hand-powered, medical instrument with an apparatus (100) according to one of claims 1 to 5, **characterized in that** the energy-generating magnet (5) is rotatably arranged in the energy detection coil (4) and induces energy therein by a rotary movement.

9. The hand-operated and hand-powered, medical instrument with an apparatus (100) according to claim 8, **characterized in that** a transmission gearing is arranged to adjust the rotational speed of the rotary movement.

10. The hand-operated and hand-powered, medical instrument with an apparatus (100) according to claim 8 or 9, **characterized in that** a flywheel mass (9) is arranged to assist in maintaining the rotary movement.

11. The hand-operated and hand-powered, medical instrument with an apparatus (100) according to one of the claims 3 to 10, **characterized in that** the memory and processing device (1) comprises an EEPROM and an integrated circuit or is formed as a combination component, and in conjunction with the magnetic core (2) and the coil (3) wound thereon provides an externally addressable and/or readable RFID/NFC apparatus with RFID/NFC functionality.

12. The hand-operated and hand-powered, medical instrument with an apparatus (100) according to one of the preceding claims, **characterized by** a smoothing buffer module (7) with charging electronics, wherein the smoothing buffer module (7) comprises a capacitor, a PowerCap, and/or a battery and is provided to support the memory and processing device (1).

## Revendications

1. Instrument médical à actionnement manuel et à commande manuelle avec un dispositif (100) pour la détection de cycles d'actionnement de l'instrument à actionnement manuel avec au moins une première et une seconde parties mobiles, dans lequel la première et la seconde partie mobile sont mobiles l'une par rapport à l'autre dans un cycle d'actionnement, dans lequel le dispositif (100) comprend ce qui suit :
une bobine de détection d'énergie (3, 4) qui est disposée au niveau d'une de la première et de la seconde parties mobiles et est disposée afin de détecter de l'énergie produite par induction ;
un aimant de production d'énergie (5) qui est disposé au niveau de l'autre de la première et de la seconde parties mobiles et est disposé afin de produire par induction de l'énergie à détecter par la bobine de détection d'énergie (3, 4),
dans lequel l'aimant de production d'énergie (5) et la bobine de détection d'énergie (3, 4) sont disposés de telle manière que l'aimant de production d'énergie (5) soit mobile lors d'un mouvement de la première et de la seconde parties mobiles l'une par rapport à l'autre dans une zone de détection de la bobine de détection d'énergie (3, 4) par rapport à celle-ci et induit de l'énergie dans celle-ci par le biais de la bobine de détection d'énergie (3, 4) ; et
un appareil de stockage et de traitement (1) qui présente un compteur et est disposé afin de détecter, pour chaque mouvement relatif, un signal de tension se basant sur l'énergie induite dans la bobine de détection d'énergie (3, 4) et d'augmenter de un le compteur pour chaque détection du signal de tension et de compter ainsi le nombre de cycles d'actionnement réalisés de l'instrument.

2. Instrument médical à actionnement manuel et à commande manuelle avec un dispositif (100) selon la revendication 1, **caractérisé en ce que** l'instrument n'est pas alimenté en énergie, en particulier en énergie d'une source d'énergie externe.

3. Instrument médical à actionnement manuel et à commande manuelle avec un dispositif selon la revendication 1, **caractérisé en ce qu'**un noyau magnétique (2) est disposé au niveau du dispositif (100) ; et la bobine de détection d'énergie (3) est enroulée sur le noyau magnétique (2).

4. Instrument médical à actionnement manuel et à commande manuelle avec un dispositif (100) selon la revendication 1, **caractérisé en ce qu'**un noyau magnétique (2) est disposé au niveau du dispositif (100), la bobine de détection d'énergie (4) est disposée séparément du noyau magnétique (2) et le noyau magnétique (2) présente une autre bobine (3) enroulée sur celui-ci.

5. Instrument médical à actionnement manuel et à commande manuelle avec un dispositif (100) selon l'une quelconque des revendications 1 à 4, **caractérisé en ce que** l'aimant de production d'énergie (5) est un aimant néodyme ou un aimant cobalt-samarium et est entouré d'un blindage (8) de telle manière que son champ magnétique soit dirigé dans un sens actif vers la bobine de détection d'énergie (4) et soit affaibli dans d'autres sens que le sens actif.

6. Instrument médical à actionnement manuel et à commande manuelle avec un dispositif (100) selon l'une quelconque des revendications 1 à 5, **caractérisé en ce que** l'aimant de production d'énergie (5) peut être introduit dans la bobine de détection d'énergie (4) et induit de l'énergie dans celle-ci par un mouvement linéaire.

7. Instrument médical à actionnement manuel et à commande manuelle avec un dispositif (100) selon la revendication 6, **caractérisé en ce que** l'aimant de production d'énergie (5) pouvant être introduit dans la bobine de détection d'énergie (4) est disposé au niveau d'un bras oscillant (10) et peut être amené en oscillation par un corps (11) sollicitant mécaniquement le bras oscillant (10).

8. Instrument médical à actionnement manuel et à commande manuelle avec un dispositif (100) selon l'une quelconque des revendications 1 à 5, **caractérisé en ce que** l'aimant de production d'énergie (5) peut être disposé de manière rotative dans la bobine de détection d'énergie (4) et induit de l'énergie dans celle-ci par un mouvement de rotation.

9. Instrument médical à actionnement manuel et à commande manuelle avec un dispositif (100) selon la revendication 8, **caractérisé en ce qu'**un engrenage de transmission est disposé pour l'adaptation de la vitesse de rotation du mouvement de rotation.

10. Instrument médical à actionnement manuel et à commande manuelle avec un dispositif (100) selon la revendication 8 ou 9, **caractérisé en ce qu'**une masse d'inertie (9) est disposée pour soutenir le maintien du mouvement de rotation.

11. Instrument médical à actionnement manuel et à commande manuelle avec un dispositif (100) selon l'une quelconque des revendications 3 à 10, **caractérisé en ce que** l'appareil de stockage et de traitement (1) présente une EEPROM et un circuit intégré ou est formé comme un module combiné, et fournit en liaison avec le noyau magnétique (2) et la bobine (3) enroulée sur celui-ci un dispositif RFID/NFC lisible et/ou accessible en externe avec une fonctionnalité RFID/NFC.

12. Instrument médical à actionnement manuel et à commande manuelle avec un dispositif (100) selon l'une quelconque des revendications précédentes, **caractérisé par** un module tampon de lissage (7) avec une électronique de charge, dans lequel le module tampon de lissage (7) présente un condensateur, un PowerCap et/ou une batterie et est fourni pour soutenir l'appareil de stockage et de traitement (1).
